# DEMANDE DE BREVET EUROPEEN

(11) **EP 2 272 491 A1**
(43) Date de publication de la demande: **12.01.2011**
(21) Numéro de dépôt: 09007987.2
(22) Date de dépôt: 18.06.2009
(51) Int. Cl.: A61K 8/35, A61L 9/14, C07C 49/207, C07C 49/217

(54) **Nouvelles compositions désodorisantes et produits désodorisants les renfermant**

(71) Demandeur: Robertet S.A., 06130 Grasse (FR)
(72) Inventeur: Casazza, André, 06130 Grasse (FR); Kerverdo, Raymond, 06460 St. Vallier de Thiey (FR); Ziegler, Lydia, 06130 Grasse (FR)
(74) Mandataire: Santarelli

(57) **Abrégé**

L'invention concerne de nouvelles compositions désodorisantes renfermant au moins un composé de la famille des cétones acétyléniques et les produits désodorisants les renfermant. Une composition particulièrement préférée selon l'invention comprend au moins un composé de la famille des cétones α-acétyléniques et un mélange d'aldéhydes choisis parmi deux familles différentes.

## Description

La présente demande concerne de nouvelles compositions désodorisantes renfermant au moins un composé de la famille des cétones acétyléniques et les produits désodorisants les renfermant.

La lutte contre les mauvaises odeurs a conduit à utiliser de nombreuses substances de grande variété, par exemple les substances phénoliques, des huiles essentielles, des résines, des aldéhydes ou des cétones, des dérivés alcooliques, des esters ou autres.

On a notamment utilisé divers aldéhydes, seuls ou en mélange avec de nombreux autres produits. Bien que donnant satisfaction, ces produits ne présentent pas toujours l'efficacité désirée.

Il existe donc toujours un besoin en composition désodorisante plus efficaces et éventuellement d'une odeur agréable.

Après de longs travaux, la Demanderesse a découvert que des molécules de la famille des cétones acétyléniques peuvent être utilisées dans des compositions désodorisantes, particulièrement du fait de leur propriété destructrice des molécules odorantes.

Ainsi l'invention a pour objet premier une composition désodorisante **caractérisée en ce qu**'elle comprend au moins un composé de la famille des cétones α-acétyléniques répondant à la formule générale 1

**R-CO-C≡C-R₁** **1**

dans laquelle
R et R₁ peuvent représenter, indépendamment ou simultanément, un radical choisi parmi
une chaine alkyle comprenant de 1 à 9, préférentiellement de 3 à 7 atomes de carbone, linéaire ou ramifiée, substituée ou non, ou
un cycloalcane comprenant de 3 à 8, préférentiellement de 5 ou 6 atomes de carbone, substitué ou non ; ou
un cycle furanique, saturé ou non, substitué ou non ; ou
un cycle pyranique, saturé ou non, substitué ou non ; ou
un cycle aromatique comprenant de 6 à 8 atomes de carbone, substitué ou non ;
R₁ pouvant en outre être un atome d'hydrogène.

Selon l'invention on entend par chaine alkyle comprenant de 1 à 9 atomes de carbone, un radical méthyle, éthyle, propyle, butyle, pentyle, hexyle, heptyle, octyle nonyle, linéaire ou ramifié, substitué ou non, en particulier par un groupement oxo, sans toutefois que le total dans la molécule n'excède 13 atomes de carbone.

Selon encore l'invention on entend par un cycloalcane comprenant de 3 à 8 atomes de carbone, un radical cyclopropyle, cyclobutyle, cyclopentyle, cyclohexyle, cycloheptyle, cyclooctyle, cyclopropylcarbonyle, cyclobutylcarbonyle, cyclopropylméthyle ou encore cyclobutylméthyle.

Selon aussi l'invention on entend par un cycle aromatique comprenant de 6 à 8 atomes de carbone, un radical choisi parmi le phényle, benzyle ou phénétyle, éventuellement substitué par un ou deux substituants, identiques ou différents, choisis parmi les substituants tolyle, xylyle, anisyle, hydroxyphényle, acétylephényle ou cyanophényle ;

Selon l'invention, les radicaux R et R₁ peuvent être, simultanément ou indépendamment substitués par un ou plusieurs radicaux choisis parmi les radicaux méthyle, éthyle, propyle, butyle ou encore un radical oxo.

Avantageusement selon l'invention, R et R1 peuvent être préférentiellement un radical acétonyle, pentyle, hexyle, heptyle ou phényle et R₁ peut être un atome d'hydrogène.

Selon l'invention, le composé de formule 1 peut être dans la composition en une quantité comprise entre 0,1% et 40%, préférentiellement entre 0,1% et 10%, en poids total de la composition.

Selon l'invention, la composition peut comprendre un ou plusieurs composés répondant à la formule 1.

Selon l'invention, on pourra utiliser préférentiellement dans les compositions au moins un des composé choisi parmi
- la 1-phényl-1-hexyne-3,5-dione de formule C₆H₅-C≡C-CO-CH₂-CO-CH₃
- la 1-phényl-1-butyne-3-one de formule C₆H₅-C≡C-CO-CH₃
- la 3-octyne-2-one de formule C₄H₉-C≡C-CO-CH₃
- la 5-décyne-2,4-dione de formule C₄H₉-C≡C-CO-CH₂-CO-CH₃
- la 5-décyne-4-one de formule C₄H₉-C≡C-CO-C₃H₇
- la 1-octyne-3-one de formule C₅H₁₁-CO-C≡CH
- la 1-phényl-2-propyne-1-one de formule C₆H₅ -CO-C≡CH
- la 4-décyne-3-one de formule C₅H₁₁-C≡C-CO-C₂H₅
- la 1-décyne-3-one de formule C₇H₁₅-CO-C≡CH

Très préférentiellement on pourra utiliser
- la 1-octyne-3-one,
- la 1-phényl-2-propyne-1-one,
- la 5-décyne-4-one ou
les couples
- 1-phényl-2-propyne-1-one/1-phényl-1-butyne-3-one ou
- 1-phényl-2-propyne-1-one/1-phényl-1-hexyne-3,5-dione.

Ces composés sont connus pour leur grande réactivité avec les thiols et les amines.

Ils sont aisément obtenus par les voies de synthèse habituelles en chimie et particulièrement
- soit par action d'un chlorure d'acide sur un dérivé sodé d'un alcyne selon

   R-COCI + NaC≡C-R₁ → R-CO-C≡C-R₁
- soit par oxidation d'un précurseur alcool secondaire selon

   R-CH(OH)-C≡C-R₁ → R-CO-C≡C-R₁

Selon une variante de l'invention, la composition désodorisante peut en outre comprendre au moins un aldéhyde choisi parmi les aldéhydes aliphatiques acycliques et non terpéniques, les aldéhydes alicycliques non terpéniques, les aldéhydes terpéniques, les aldéhydes aliphatiques substitués par un groupe aromatique, les aldéhydes bifonctionnels, les aldéhydes possédant une insaturation non aromatique portée par le carbone en alpha de la fonction aldéhyde, les aldéhydes possédant une insaturation en alpha de la fonction aldéhyde conjuguée avec un cycle aromatique et les aldéhydes dont la fonction est portée par un cycle aromatique. Dans cette variante, la composition peut comprendre un mélange de deux ou plusieurs aldéhydes.

Selon une autre variante de l'invention, préférée, la composition désodorisante peut comprendre en outre un mélange d'au moins un premier aldéhyde (aldéhyde de classe A) choisi parmi les aldéhydes aliphatiques acycliques et non terpéniques, les aldéhydes alicycliques non terpéniques, les aldéhydes terpéniques, les aldéhydes aliphatiques substitués par un groupe aromatique et les aldéhydes bifonctionnels et d'au moins un second aldéhyde (aldéhyde de classe B) choisi parmi les aldéhydes possédant une insaturation non aromatique portée par le carbone en alpha de la fonction aldéhyde, les aldéhydes possédant une insaturation en alpha de la fonction aldéhyde conjuguée avec un cycle aromatique et les aldéhydes dont la fonction est portée par un cycle aromatique. Dans cette variante, la composition peut comprendre un mélange d'au moins un premier et d'au moins un second aldéhyde. Mais bien évidement le premier aldéhyde peut être un mélange de deux ou plusieurs aldéhydes de classe A et le second aldéhyde peut être un mélange de deux ou plusieurs aldéhydes choisis parmi ceux de classe B.

Selon l'invention, l'aldéhyde aliphatique acyclique et non terpénique de classe A peut être choisi parmi
- le décanal,
- l'undécanal,
- le dodécanal,
- l'undécène-10-al,
- le méthyl-2-undécanal,
- le triméthyl-2,6,10-undécène-9-al (ADOXAL™), ou encore
- le tétraméthyl-2,3,5,5-hexanal.

Préférentiellement l'aldéhyde aliphatique acyclique et non terpénique (AANT) pourra être choisi parmi les aldéhydes naturels ou identiques nature, non classés dangereux pour l'environnement selon la directive Européenne «Substances dangereuses»., autorisés en alimentaire (n°FEMA) et en parfumerie par l'IFRA (cette remarque est valable pour le choix d'ensemble des aldéhydes des différentes classes. La liste des aldéhydes aliphatiques de classe A et B répondant aux critères ci-dessus (alimentaires, non classés...) est très longue.

Encore selon l'invention, l'aldéhyde alicyclique non terpénique de classe A peut être choisi parmi
- le 2,4-diméthylcyclohex-3-ene-1-carboxaldéhyde,
- le 1,2-diméthylcyclohex-3-ene-1-carboxaldehyde,
- le 3,5-diméthylcyclohex-3-ene-1-carboxaldehyde,
- le 2,4,6-triméthylcyclohex-3-ene-1-carboxaldehyde (ISOCYCLOCITRAL™),
- le tricyclo[5.2.1.0(0.6)]décylidène-8)-4-butanal (DUPICAL™),
- le triméthyl-2,6,10- undécène-9 al (ADOXAL™),
- le (méthyl-4-pentène-3-yl)-4-cyclohexène-3-yl-1-carboxaldéhyde,
- le 6-méthyl-8-(1-méthyléthyl)bicyclo[2.2.2]-5-octene-2-carboxaldehyde (MACEAL™) ou encore
- le 8,8-diméthyl-2,3,4,5,6,7-hexahydro-1H-naphthalene-2-carboxaldehyde (ALDEHYDE 111™).

Préférentiellement l'aldéhyde alicyclique non terpénique (AliNT) pourra être
- le 8,8-diméthyl-2,3,4,5,6,7-hexahydro-1H-naphthalene-2-carboxaldéhyde (ALDEHYDE 111™),
- le 5 méthyl furfural,
- le 1-méthyl-4-(4-méthylpentyl)cyclohex-3-ene-1-carbaldehydecarboxaldéhyde,
- le 3-(5-méthyl-2-furyl)butanal,6,6-diméthylbicyclo[3.1.1]-2-heptenyl-2-propanal,
- le tricyclodecanylcarboxaldehyde,
- le butylcinnamaldehyde.

L'aldéhyde terpénique (Terp) de classe A peut être choisi parmi
- le citronellal, l'hydroxycitronnellal,
- le 2,2,3-triméthyl-3-cyclopentène-1-acetaldéhyde (aldéhyde campholénique),
- le p-menth-1-ène-9-al (carvomenthènal),
- le 7,7-diméthylbicyclo[3.1.1]hept-3-ène-4-carboxaldéhyde (myrtenal),
- le 2-isopropyl-5-méthyl-2-hexènal (isodihydrolavandulal). Préférentiellement selon l'invention, l'aldéhyde terpénique peut être
- l'aldéhyde campholénique,
- le carvomenthenal,
- le myrtènal ou
- l'isodihydrolavandulal.

L'aldéhyde aliphatique de classe A substitué par un groupe aromatique peut être choisi parmi
- le 2-méthyl-3-(p-isopropylphényl)propanal,
- le 3-(4-tert-butylphényl)butanal,
- l'aldéhyde phénylacétique,
- le 3-phénylpropanal,
- le 2-phénylpropanal (aldéhyde hydratropique),
- le 2-méthyl-3-(p-méthylphényl)propanal,
- l'hélional^{™},
- l'aldéhyde cyclamen,
- le lilial,
- le canthoxal^{™},
- l'aldéhyde phénylacétique,
- le 3-phénylpropanal,
- l'aldéhyde hydratropique.

Préférentiellement selon l'invention, l'aldéhyde aliphatique substitué par un groupe aromatique peut être
- le 2-méthyl-3-(p-isopropylphényl)propionaldéhyde,
- le 3-(4-tert-butylphényl)butanal,
- l'aldéhyde phénylacétique, le 3-phénylpropanal,
- le 2-phenylpropanal (aldéhyde hydratropique),
- le 2-méthyl-3-(p-méthylphényl)propanal.

Par "aldéhyde bifonctionnel", on entend selon l'invention les aldéhydes possédant par ailleurs une autre fonction comme par exemple une fonction éther-oxyde ou alcool. L'aldéhyde bifonctionnel peut être selon l'invention choisi parmi
- les alcoxy-acétaldéhydes,
- les ω-hydroxy-aldéhydes (hydroxycitronellal),
- les 3 et 4-(4-hydroxy-4-méthylpentyl)-3-cyclohexène-1-carboxaldéhyde (LYRAL™),
- les ω-alcoxy-aldéhydes,
- le méthoxydicyclopentadiènecarboxyaldéhyde,
- le 3-(1,3-benzodioxol-5-yl)-2-méthylpropanal,
- le 7-hydroxy-3,7-diméthyloctanal.

Préférentiellement selon l'invention, l'aldéhyde bifonctionnel peut être
- le méthoxydicyclopentadiènecarboxyaldehyde, ou
- le 7-hydroxy-3,7-diméthyloctanal.

Selon l'invention, l'aldéhyde qui possède une insaturation de type non aromatique (classe B) portée par le carbone en position alpha de la fonction aldéhyde peut être choisi parmi
- le citral (néral et géranial),
- le myrténal,
- l'aldéhyde périllique,
- les furyl-2-carboxaldéhydes diversement substitués,
- le 2,4-heptadiènal,
- le trans-2, trans-4-undecadiènal,
- le trans-2 trans-4-dodecadiènal,
- le trans-2-décènal,
- le trans-2,4-cis, cis-7-tridecatriènal,
- le trans-2, cis-6-nonadiènal,
- le 3,7-diméthyl-2-méthylène-6-octènal,
- le trans-2-dodécènal.

Préférentiellement selon l'invention, l'aldéhyde qui possède une insaturation de type non aromatique portée par le carbone en position alpha de la fonction aldéhyde peut être
- le citral,
- le myrténal,
- l'aldéhyde périllique,
- le trans-2-dodécènal,
- le 2-furfurylidènebutyraldéhyde.

L'aldéhyde (de classe B) qui possède une insaturation éthylénique en alpha, conjuguée avec un cycle aromatique peut être choisi parmi
- l'aldéhyde cinnamique,
- l'aldéhyde α-amylcinnamique (JASMONAL™),
- l'aldéhyde α-hexylcinnamique ou encore
- le (E)-3-(2-méthoxyphényl)prop-2-ènal,
- le (E)-3-(4-méthoxyphényl)prop-2-ènal,
- l'α-méthylcinnamaldéhyde,
- le 1-benzofurane-2-carboxaldéhyde,
- le 2-phenyl-2-butènal,
- le 5-méthyl-2-phényl-2-hexènal.

Préférentiellement selon l'invention, l'aldéhyde qui possède une insaturation éthylénique en alpha, conjuguée avec un cycle aromatique peut être
- le (E)-3-(2-méthoxyphényl)prop-2-ènal,
- le (E)-3-(4-méthoxyphényl)prop-2-ènal,
- le 1-benzofurane-2-carboxaldéhyde,
- le 2-phenyl-2-butènal,
- le 5-méthyl-2-phenyl-2-hexènal.

L'aldéhyde porté par un cycle aromatique, par ailleurs diversement substitués, peut être choisi parmi
- l'aldéhyde cinnamique,
- l'aldéhyde α-amylcinnamique (JASMONAL™),
- l'aldéhyde α-hexylcinnamique ou encore
- le (E)-3-(2-méthoxyphényl)prop-2-ènal,
- le (E)-3-(4-méthoxyphényl)prop-2-ènal,
- l'α-méthylcinnamaldéhyde,
- le 1-benzofurane-2-carboxaldéhyde,
- le 2-phenyl-2-butènal,
- le 5-méthyl-2-phényl-2-hexènal.

Préférentiellement selon l'invention, l'aldéhyde porté par un cycle aromatique, par ailleurs diversement substitués, peut être
- la vanilline,
- l'éthylvanilline,
- l'acétate de vanilline,
- le benzaldéhyde,
- l'aldéhyde anisique.

Dans des conditions préférentielles de mise en oeuvre de la seconde variante de l'invention ci-dessus décrite, le premier aldéhyde peut être choisi de préférence parmi les aldéhydes aliphatiques acycliques et non terpéniques, les aldéhydes alicycliques non terpéniques, les aldéhydes terpéniques et les aldéhydes aliphatiques substitués par un groupe aromatique, le second aldéhyde peut être choisi de préférence parmi les aldéhydes qui possèdent une insaturation de type non aromatique portée par le carbone alpha.

Tout particulièrement, le premier aldéhyde peut être choisi parmi les aldéhydes aliphatiques ou acycliques et non terpéniques et le second parmi les aldéhydes terpéniques possédant une insaturation sur le carbone alpha ci-dessus désignés.

Des couples d'aldéhydes particulièrement intéressants peuvent être notamment les couples suivants :
- le dodécanal et le myrténal,
- le dodécanal et le citral,
- l'adoxal et l'aldéhyde périllique,
- le triplal et le citral,
- le macéal et le citral,
- l'adoxal et le myrténal,
- le décanal et le citral,
- l'undécène-10-al et le myrténal,
ainsi que les couples de produits cités dans les exemples.

Selon la première variante de l'invention la composition peut comprendre l'aldéhyde en une quantité comprise entre 0,5% et 50% de la composition, préférentiellement en une quantité comprise entre 1 % et 20% de la composition.

Selon la seconde variante de l'invention la composition peut comprendre les aldéhydes des classes A et B en proportions relatives l'un par rapport à l'autre en proportions de 80/20 à 20/80 notamment en proportions de 50/50 et dans une quantité totale d'aldéhydes comprise entre 1 % et 90 % de la composition, préférentiellement en une quantité comprise entre 10% et 30% de la composition.

Les compositions désodorisantes selon l'invention, en plus de leurs remarquables propriétés désodorisantes, possèdent en outre par elles-mêmes des propriétés odoriférantes capables de remplacer les mauvaises odeurs par leur propre odeur.

Selon encore une autre variante de l'invention les compositions désodorisantes peuvent en outre comprendre un agent masquant.

Ces compositions peuvent donc comporter au moins un agent odoriférant tel que ceux habituellement utilisés en parfumerie comme des alcools, huiles essentielles, substances phénoliques, esters.

Ces compositions désodorisantes trouvent leur application dans toutes les conditions où la lutte contre les mauvaises odeurs est recherchée, quelle que soit leur origine.

Les compositions selon l'invention peuvent renfermer en outre des substances spécifiques, comme par exemple des bactéricides.

Ces compositions désodorisantes sont avantageusement formulées selon les techniques habituelles.

C'est pourquoi la présente demande a également pour objet les produits désodorisants **caractérisés en ce qu**'ils renferment les compositions ci-dessus décrites.

Ces produits peuvent se présenter sous forme de pulvérisateurs aérosols, supports solides imprégnés, liquides, crèmes, poudres, etc. Ils peuvent être réalisés selon les méthodes connues de l'homme de l'art.

L'invention a encore pour objet l'utilisation d'un composé de la famille des cétones acétyléniques répondant à la formule 1 dans une composition désodorisante, particulièrement dans des compositions désodorisantes comprenant au moins un aldéhyde choisi parmi les aldéhydes aliphatiques acycliques et non terpéniques, les aldéhydes alicycliques non terpéniques, les aldéhydes terpéniques, les aldéhydes aliphatiques substitués par un groupe aromatique, les aldéhydes bifonctionnels, les aldéhydes possédant une insaturation non aromatique portée par le carbone en alpha de la fonction aldéhyde, les aldéhydes possédant une insaturation en alpha de la fonction aldéhyde conjuguée avec un cycle aromatique et les aldéhydes dont la fonction est portée par un cycle aromatique, et encore plus préférentiellement dans une composition désodorisante comprenant un mélange d'au moins un premier aldéhyde (aldéhyde de classe A) choisi parmi les aldéhydes aliphatiques acycliques et non terpéniques, les aldéhydes alicycliques non terpéniques, les aldéhydes terpéniques, les aldéhydes aliphatiques substitués par un groupe aromatique et les aldéhydes bifonctionnels et d'au moins un second aldéhyde (aldéhyde de classe B) choisi parmi les aldéhydes possédant une insaturation non aromatique portée par le carbone en alpha de la fonction aldéhyde, les aldéhydes possédant une insaturation en alpha de la fonction aldéhyde conjuguée avec un cycle aromatique et les aldéhydes dont la fonction est portée par un cycle aromatique.

### Partie expérimentale

Les exemples qui suivent illustrent la présente invention, sans toutefois la limiter.

### Préparation des cétones α-acétyléniques

### I- Cétones acétyléniques disubstituées R-CO-C≡C-R₁

### Préparation de la 4-décyne-3-one (atmosphère inerte)

Dans un réacteur de 1 litre on charge 345 ml de diglyme (RN 111-96-6) et 17,2g (748 mmoles) de sodium (RN 7440-23-5) coupé en morceaux. On chauffe à une température un peu supérieure à la fusion du sodium et, sous bonne agitation, on ajoute alors en quatre fois à 1 h d'intervalle 71,7g (745 mmoles) soit 97,5 ml de 1-heptyne (RN 628-71-7) en solution dans 100 ml de diglyme. Le milieu s'épaissit pour former une suspension beige. On agite encore pendant 2h30 à la même température. On refroidit l'ensemble à 25-30°C et on le verse lentement sur une solution agitée de 64,8g (700 mmoles) soit 61,0 ml de chlorure de propionyle (RN 79-03-8) dans 600 ml d'éther éthylique. Une réaction exothermique se produit. On agite ensuite pendant 1 h sans s'occuper de la température.

On lave par 4 x 1 L d'eau, on sèche et concentre sous pression réduite (25 mm) pour obtenir 98g d'un liquide jaune orangé. Ce dernier est fractionné sous pression réduite (2 mm). On recueille la fraction qui passe à 70-1 °C. Rendement 50 g soit 47%.

De manière analogue sont préparés (exemples non limitatifs): la 5-décyne-4-one, la 3-octyne-2-one et la 1-phényl-1-butyne-3-one.

### II- Cétones acétyléniques vraies R-CO-C≡CH

### Préparation de la 1-octyne-3-one

### 1) 1-octyne-3-ol (atmosphère inerte)

Dans un réacteur de 750 ml on charge 40,1 g (400 mmoles) soit 49,2 ml d'hexanal (RN 66-25-1) et 400 ml de tétrahydrofurane anhydre et sous agitation on ajoute par portions 100g d'une suspension d'acétylure de sodium (RN 1066-26-8) à 18% dans le xylène (375 mmoles). Une réaction exothermique se produit. On agite ensuite encore pendant 2h sans s'occuper de la température. Après hydrolyse, extractions et concentration, on fractionne sous pression réduite.

Il est toutefois à noter qu'un meilleur résultat est obtenu par l'emploi de bromure d'éthynylmagnésium (RN 4301-14-8). Ainsi par exemple pour le 1-décyne-3-ol.

### 2) 1-octyne-3-one

Dans un réacteur de 1500 ml on charge 25,24g (200 mmoles) soit 29,1 ml de 1-octyne-3-ol, 600 ml d'acétone et 90 ml d'eau. Sous agitation énergique on ajoute en 1 h30 une solution de 25,0 g (85 mmoles) de bichromate de potassium (RN 7778-50-9) dans 300 ml d'eau et 18,2 ml d'acide sulfurique. La coloration passe de l'orange au vert. A la fin, on agite encore pendant 30 mn puis on ajoute 1000 ml d'eau en 30 mn également.

Après extractions à l'éther éthylique, lavages, séchage et concentration sous pression faiblement réduite, on obtient 25 g d'un liquide orange qu'on fractionne sous pression réduite (20 mm). On recueille la fraction qui passe à 74-76°C.

De manière analogue on prépare la 1-phényl-2-propyne-1-one et la 1-décyne-3-one.

### Evaluation de l'effet des compositions selon l'invention sur les mauvaises odeurs

L'effet sur les mauvaises odeurs des compositions selon la présente invention a été évalué à l'aide des dispositifs suivants :

Utilisation de cabine d'olfaction

La mauvaise odeur de toilette, formulée selon la méthode de préparation décrite ci-après est versée dans la cuvette des WC.

On pulvérise dans la cuvette des WC, un spray aqueux parfumé avec un neutralisant «magnolia» contenant les aldéhydes suivants :

| | |
|---|---|
| Ald. phénylacétique (A) | 0,318182% |
| Hydroxycitronellal (A) | 1,636364% |
| Ald. α-hexylcinnamique (B) | 19,090909% |
| Lilial (A) | 7,909091% |

Une cabine témoin ne reçoit que du 2,4-diméthyl-3-cyclohexene-1-carboxaldéhyde (A) à 0,154545%.

Le neutralisant magnolia contenait ou non 0,1% de cétone(s) acétylénique(s)

L'effet neutralisant le plus net et le plus fort, non seulement immédiatement mais également après un délai de 5 minutes, est obtenu lorsque l'on utilise des couples d'aldéhydes appartenant à la fois aux deux classes A et B et d'un ou plusieurs composés de la famille des cétones acétyléniques

### Préparation de mauvaises odeurs standards pour évaluation sensorielle de l'efficacité neutralisante

### Odeur de toilettes

| **Matières premières** | **%** | **Odeur** |
|---|---|---|
| Scatole | 0,91 | Fécale |
| β-Thionaphtol Fluka 88910 | 0,91 | Fécale, Soufrée |
| Acide Thioglycolique Fluka 88650 | 21,18 | Soufrée |
| Acide Caproïque | 6,00 | Transpiration |
| Phényle acétate p-crésyl | 1,00 | |
| p-Cresyl isovalerate | / | Fécale, transpiration |
| N-Méthyl morpholine | / | Urine |
| Dipropylene glycol | qsp 100 | |

Dosage : 2 ml de la solution à 1 % sur une ouate de cellulose de 10 cm² pour une pièce d'un m³.

### Odeurs de cuisine

| **Matières premières** | **%** | **Odeur** |
|---|---|---|
| Triméthylamine | 0,10 | Poisson |
| Diméthyl sulfure | 0,13 | Choux |
| Diméthyl disulfure | 0,02 | Oignon |
| Pyridine-2-méthanethiol | 0,05 | Viande putréfiée |
| Acide isovalérique | 0,70 | transpiration, fromage |
| Acide butyrique | 0,30 | fromage |
| Acide caprique | 0,50 | fromage, fermentation |
| Méthyl amyl cétone | 0,10 | Roquefort |
| Concentré marine | / | poisson, algue |
| 1-Mercapto propanone | / | viande, putréfaction, moisie |
| Diallyl sulfure | 0,05 | ail |
| Butyl butyrolactate | 1,30 | lait caillé |
| Methonional | 0,02 | vielle pomme de terre |
| Indole à 1% | 0,14 | légumes moisis, pourris |
| Diacétyle | 0,10 | beurre rance |
| Acide caproïque | 0,20 | fromage trop fait |
| Guaïacol | 0,4 à 10 | viande cuite |
| Diallyl disulfure | 0,05 | |
| Base huître | 0,20 | |
| Base saumon | 0,20 | |
| Base crevette | 0,20 | |
| Dipropylene glycol | qsp 100 | |

Odeur de tabac : L'odeur de tabac est extraite en phase solvant à partir de mégots de cigarette ou produite par la combustion de cigarettes en enceinte close.

| | | |
|---|---|---|
| **Matières premières** | | **Odeur** |
| Racinol (plus commercialisé) | / | boisée, sale |
| Costus ollifac | 6,00 | animal, fécale |
| 4-Méthylthio-4-méthyl-2-pentanone | / | urine de chat |
| Acide phényle acétique | 10,00 | cheval, urine |
| Isopropyl quinoléine | / | fécale, sale |
| Scatole | 2,00 | fécale, sale |
| Aldéhyde C12 laurique | 6,00 | brulée |
| Acide isovalérique | 0,04 | transpiration |
| Acide isobutyrique | 0,04 | rance |
| Civette 170L | 1,00 | fécale, animale |
| Castoréum rectifié artificiel | 1,00 | animal |
| Diacétyle | 0,10 | beurre |
| Thiomenthone à 1 % | / | urine de chat |
| Diméthyl sulfure | 0,10 | oignon frit |
| Animalis | / | animal |
| 8-Mercapto-p-menthan-3-one | 2,00 | Urine |
| Diméthylmercaptobutanol | 0,10 | |
| Dipropylene glycol | qsp 100 | |

### 1. Aérosol désodorisant d'atmosphère

On a réalisé un spray d'ambiance aqueux parfumé avec un neutralisant «magnolia» contenant les aldéhydes suivants :

| | |
|---|---|
| Ald phenylacetique (A) | 0,318182% |
| Hydroxycitronellal (A) | 1,636364% |
| Ald αhexylcinnamique (B) | 19,090909% |
| Lilial (A) | 7,909091% |
| 2,4-diméthyl-3-cyclohexene-1-carboxaldehyde (A) | 0,154545% |

Le neutralisant magnolia contenait ou non 0,1% de cétone(s) acétylénique(s).

### 2. Désodorisant et désinfectant liquide pour sols :

Les aldéhydes peuvent également être formulés sous la forme d'acétals afin de réduire l'odeur intrinsèque du neutralisant et d'augmenter la durée d'action. Il est connu qu'un acétal s'hydrolyse en milieu acide en libérant l'aldéhyde correspondant.

Voici à titre d'exemples quelques acétals faiblement odorants pouvant être utilisés dans un désodorisant de surface : propionaldehyde DEA, phenylacetaldehyde-méthanol acétal, citral diéthylacetal, hydroxycitronnellal diméthylacetal, benzaldehyde diéthylacetal.

### Formule de désodorisant de surface :

| **%** | **Nom du produit** |
|---|---|
| 15,0 | Alcool éthylique |
| 0,5 | Sodium lauroyl sarcosinate |
| 2,0 | Sodium carbonate |
| 0,5 | Sodium dioctyl sulfosuccinate |
| 0,5 | Neutralisant à base d'aldéhydes, d'acétals et d'un parfum bactéricide |
| qsp100 | Eau déminéralisée |

## Revendications

1. Composition désodorisante **caractérisée en ce qu'**elle comprend au moins un composé de la famille des cétones α-acétyléniques répondant à la formule générale 1
**R-CO-C≡C-R₁** **1**
dans laquelle
R et R₁ peuvent représenter, indépendamment ou simultanément, un radical choisi parmi
■ une chaine alkyle comprenant de 1 à 9, préférentiellement de 3 à 7 atomes de carbone, linéaire ou ramifiée, substituée ou non, ou
■ un cycloalcane comprenant de 3 à 8, préférentiellement de 5 ou 6, atomes de carbone, substitué ou non ; ou
■ un cycle furanique, saturé ou non, substitué ou non ; ou
■ un cycle pyranique, saturé ou non, substitué ou non ; ou
■ un cycle aromatique comprenant de 6 à 8 atomes de carbone, substitué ou non ;
■ R₁ pouvant en outre être un atome d'hydrogène.

2. Composition désodorisante selon la revendication 1, **caractérisée en ce que** le composé de formule 1 est choisi parmi
- la 1-phényl-1-hexyne-3,5-dione de formule C₆H₅-C≡C-CO-CH₂-CO-CH₃
- la 1-phényl-1-butyne-3-one de formule C₆H₅-C≡C-CO-CH₃
- la 3-octyne-2-one de formule C₄H₉-C≡C-CO-CH₃
- la 5-décyne-2,4-dione de formule C₄H₉-C≡C-CO-CH₂-CO-CH₃
- la 5-décyne-4-one de formule C₄H₉-C≡C-CO-C₃H₇
- la 1-octyne-3-one de formule C₅H₁₁-CO-C≡CH
- la 1-phényl-2-propyne-1-one de formule C₆H₅-CO-C≡CH
- la 4-décyne-3-one de formule C₅H₁₁C≡C-CO-C₂H₅
- la 1-décyne-3-one de formule C₇H₁₅-CO-C≡CH

3. Composition désodorisante selon l'une quelconque des revendications 1 ou 2, **caractérisée en ce que** le composé de formule 1 est dans la composition en une quantité comprise entre 0,1% et 40%, préférentiellement entre 0,1% et 10%, en poids total de la composition.

4. Composition désodorisante selon l'une quelconque des revendications 1 à 3, **caractérisée en ce qu'**elle comprend en outre au moins un aldéhyde choisi parmi les aldéhydes aliphatiques acycliques et non terpéniques, les aldéhydes alicycliques non terpéniques, les aldéhydes terpéniques, les aldéhydes aliphatiques substitués par un groupe aromatique, les aldéhydes bifonctionnels, les aldéhydes possédant une insaturation non aromatique portée par le carbone en alpha de la fonction aldéhyde, les aldéhydes possédant une insaturation en alpha de la fonction aldéhyde conjuguée avec un cycle aromatique et les aldéhydes dont la fonction est portée par un cycle aromatique.

5. Composition désodorisante selon l'une quelconque des revendications 1 à 3, **caractérisée en ce qu'**elle comprend en outre un mélange d'au moins un premier aldéhyde (aldéhyde de classe A) choisi parmi les aldéhydes aliphatiques acycliques et non terpéniques, les aldéhydes alicycliques non terpéniques, les aldéhydes terpéniques, les aldéhydes aliphatiques substitués par un groupe aromatique et les aldéhydes bifonctionnels et d'au moins un second aldéhyde (aldéhyde de classe B) choisi parmi les aldéhydes possédant une insaturation non aromatique portée par le carbone en alpha de la fonction aldéhyde, les aldéhydes possédant une insaturation en alpha de la fonction aldéhyde conjuguée avec un cycle aromatique et les aldéhydes dont la fonction est portée par un cycle aromatique.

6. Composition désodorisante selon la revendication 4, **caractérisée en ce qu'**elle comprend une quantité de l'aldéhyde comprise entre 0,5% et 50% de la composition, préférentiellement en une quantité comprise entre 1% et 20% de la composition.

7. Composition désodorisante selon la revendication 5, **caractérisée en ce que** les aldéhydes des classes A et B sont en proportions relatives l'un par rapport à l'autre en proportions de 80/20 à 20/80 notamment en proportions de 50/50 et dans une quantité totale d'aldéhydes comprise entre 1 % et 90 % de la composition, préférentiellement en une quantité comprise entre 10% et 30% de la composition.

8. Composition désodorisante selon l'une quelconque des revendications 4 à 7, **caractérisée en ce que** l'aldéhyde aliphatique acyclique de classe A et non terpénique peut être choisi parmi
- le décanal,
- l'undécanal,
- le dodécanal,
- l'undécène-10-al,
- le méthyl-2-undécanal,
- le triméthyl-2,6,10-undécène-9-al (ADOXAL™), ou encore
- le tétraméthyl-2,3,5,5-hexanal.

9. Composition désodorisante selon l'une quelconque des revendications 4 à 7, **caractérisée en ce que** l'aldéhyde alicyclique non terpénique de classe A est choisi parmi
- le 2,4-diméthylcyclohex-3-ene-1-carboxaldéhyde,
- le 1,2-diméthylcyclohex-3-ene-1-carboxaldehyde,
- le 3,5-diméthylcyclohex-3-ene-1-carboxaldehyde,
- le 2,4,6-triméthylcyclohex-3-ene-1-carboxaldehyde (ISOCYCLOCITRAL™),
- le tricyclo[5.2.1.0(0.6)]décylidène-8)-4-butanal (DUPICAL™),
- le triméthyl-2,6,10- undécène-9 al (ADOXAL™),
- le (méthyl-4-pentène-3-yl)-4-cyclohexène-3-yl-1-carboxaldéhyde,
- le 6-méthyl-8-(1-méthyléthyl)bicyclo[2.2.2]-5-octene-2-carboxaldehyde (MACEAL™) ou encore
- le 8,8-diméthyl-2,3,4,5,6,7-hexahydro-1H-naphthalene-2-carboxaldehyde (ALDEHYDE 111™)_{.}

10. Composition désodorisante selon l'une quelconque des revendications 4 à 7, **caractérisée en ce que** l'aldéhyde terpénique de classe A est choisi parmi
- le citronellal, l'hydroxycitronnellal,
- le 2,2,3-triméthyl-3-cyclopentène-1-acetaldéhyde (aldéhyde campholénique),
- le p-menth-1-ène-9-al (carvomenthènal),
- le 7,7-diméthylbicyclo[3.1.1]hept-3-ène-4-carboxaldéhyde (myrtènal),
- le 2-isopropyl-5-méthyl-2-hexènal (isodihydrolavandulal).

11. Compositions désodorisantes selon l'une quelconque des revendications 4 à 7, **caractérisées en ce que** l'aldéhyde aliphatique de classe A substitué par un groupe aromatique est choisi parmi
- le 2-méthyl-3-(p-isopropylphényl)propional,
- le 3-(4-tert-butylphényl)butanal,
- l'aldéhyde phénylacétique,
- le 3-phénylpropanal,
- le 2-phénylpropanal (aldéhyde hydratropique),
- le 2-méthyl-3-(p-méthylphényl)propanal,
- l'hélional^{™},
- l'aldéhyde cyclamen,
- le lilial,
- le canthoxal^{™},
- l'aldéhyde phénylacétique,
- le 3-phénylpropanal,
- l'aldéhyde hydratropique.

12. Compositions désodorisantes selon l'une quelconque des revendications 4 à 7, **caractérisées en ce que** l'aldéhyde bifonctionnel est choisi parmi les alcoxy-acétaldéhydes,
- les ω-hydroxy-aldéhydes (hydroxycitronellal),
- les 3 et 4-(4-hydroxy-4-méthylpentyl)-3-cyclohexène-1-carboxaldéhyde (LYRAL^{™}),
- les ω-alcoxy-aldéhydes,
- le méthoxydicyclopentadiènecarboxyaldéhyde,
- le 3-(1,3-benzodioxol-5-yl)-2-méthylpropanal,
- le 7-hydroxy-3,7-diméthyloctanal.

13. Compositions désodorisantes selon l'une quelconque des revendications 4 à 7, **caractérisées en ce que** l'aldéhyde qui possède une insaturation de type non aromatique (classe B) portée par le carbone en position alpha de la fonction aldéhyde est choisi parmi
- le citral (néral et géranial),
- le myrténal,
- l'aldéhyde périllique,
- les furyl-2-carboxaldéhydes diversement substitués,
- le 2,4-heptadiènal,
- le trans-2, trans-4-undecadiènal,
- le trans-2 trans-4-dodecadiènal,
- le trans-2-décènal,
- le trans-2,4-cis, cis-7-tridecatriènal,
- le trans-2, cis-6-nonadiènal,
- le 3,7-diméthyl-2-méthylène-6-octènal,
- le trans-2 dodécènal.

14. Compositions désodorisantes selon l'une quelconque des revendications 4 à 7, **caractérisées en ce que** l'aldéhyde (de classe B) qui possède une insaturation éthylénique en alpha, conjuguée avec un cycle aromatique est choisi parmi
- l'aldéhyde cinnamique,
- l'aldéhyde α-amylcinnamique (JASMONAL™),
- l'aldéhyde α-hexylcinnamique,
- le (E)-3-(2-méthoxyphényl)prop-2-ènal,
- le (E)-3-(4-méthoxyphényl)prop-2-ènal,
- l'α-méthylcinnamaldéhyde,
- le 1-benzofurane-2-carboxaldéhyde,
- le 2-phenyl-2-butènal,
- le 5-méthyl-2-phényl-2-hexènal.

15. Compositions désodorisantes selon l'une quelconque des revendications 4 à 7, **caractérisées en ce que** l'aldéhyde porté par un cycle aromatique, par ailleurs diversement substitués, est choisi parmi
- l'aldéhyde cinnamique,
- l'aldéhyde α-amylcinnamique (JASMONAL™),
- l'aldéhyde α-hexylcinnamique ou encore
- le (E)-3-(2-méthoxyphényl)prop-2-ènal,
- le (E)-3-(4-méthoxyphényl)prop-2-ènal,
- l'α-méthylcinnamaldéhyde,
- le 1-benzofurane-2-carboxaldéhyde,
- le 2-phenyl-2-butènal,
- le 5-méthyl-2-phényl-2-hexènal.

16. Utilisation d'un composé de la famille des cétones α-acétyléniques répondant à la formule 1
R-CO-C≡C-R₁ **1**
dans laquelle
R et R₁ peuvent représenter, indépendamment ou simultanément, un radical choisi parmi
■ une chaine alkyle comprenant de 1 à 9, préférentiellement de 3 à 7 atomes de carbone, linéaire ou ramifiée, substituée ou non, ou
■ un cycloalcane comprenant de 3 à 8, préférentiellement de 5 ou 6, atomes de carbone, substitué ou non ; ou
■ un cycle furanique, saturé ou non, substitué ou non ; ou
■ un cycle pyranique, saturé ou non, substitué ou non ; ou
■ un cycle aromatique comprenant de 6 à 8 atomes de carbone, substitué ou non ;
■ R₁ pouvant en outre être un atome d'hydrogène,
dans une composition désodorisante, particulièrement dans des compositions désodorisantes comprenant au moins un aldéhyde choisi parmi les aldéhydes aliphatiques acycliques et non terpéniques, les aldéhydes alicycliques non terpéniques, les aldéhydes terpéniques, les aldéhydes aliphatiques substitués par un groupe aromatique, les aldéhydes bifonctionnels, les aldéhydes possédant une insaturation non aromatique portée par le carbone en alpha de la fonction aldéhyde, les aldéhydes possédant une insaturation en alpha de la fonction aldéhyde conjuguée avec un cycle aromatique et les aldéhydes dont la fonction est portée par un cycle aromatique, et encore plus préférentiellement dans une composition désodorisante comprenant un mélange d'au moins un premier aldéhyde (aldéhyde de classe A) choisi parmi les aldéhydes aliphatiques acycliques et non terpéniques, les aldéhydes alicycliques non terpéniques, les aldéhydes terpéniques, les aldéhydes aliphatiques substitués par un groupe aromatique et les aldéhydes bifonctionnels et d'au moins un second aldéhyde (aldéhyde de classe B) choisi parmi les aldéhydes possédant une insaturation non aromatique portée par le carbone en alpha de la fonction aldéhyde, les aldéhydes possédant une insaturation en alpha de la fonction aldéhyde conjuguée avec un cycle aromatique et les aldéhydes dont la fonction est portée par un cycle aromatique.
